# EUROPÄISCHE PATENTSCHRIFT

(11) **EP 2 752 183 B1**
(45) Veröffentlichungstag und Bekanntmachung des Hinweises auf die Patenterteilung: **22.04.2015**
(21) Anmeldenummer: 13150370.8
(22) Anmeldetag: 07.01.2013
(51) Int. Cl.: A61K 8/26, A61K 8/29, A61Q 17/04, A61Q 19/00, A61K 8/92, A61K 8/97, A61K 8/19

(54) **Kosmetikum mit biologischem Naturstoffkomplex**
Cosmetic product with biological natural material complex
Cosmétique avec complexe biologique en matière naturelle

(43) Veröffentlichungstag der Anmeldung: 09.07.2014
(73) Patentinhaber: Golüke, Timm, 80539 München (DE)
(72) Erfinder: Golüke, Timm, 80539 München (DE)
(74) Vertreter: Gulde & Partner

(56) Entgegenhaltungen:
- DE-A1-102007 005 186
- DE-U1-202006 019 183
- FR-A1- 2 648 347
- FR-A1- 2 696 075
- FR-A1- 2 768 621
- LAI HOW Y ET AL: "Blechnum Orientale Linn - a fern with potential as antioxidant, anticancer and antibacterial agent", BMC COMPLEMENTARY AND ALTERNATIVE MEDICINE, Bd. 10, Nr. 1, 15, 30. April 2010 (2010-04-30), Seiten 1-8, XP021076632, BIOMED CENTRAL LTD., LONDON, GB ISSN: 1472-6882, DOI: 10.1186/1472-6882-10-15
- PHILIPS NEENA ET AL: "Counteraction of Skin Inflammation and Aging or Cancer by Polyphenols and Flavonoids from Polypodium leucotomos and Xanthohumol", ANTI-INFLAMMATORY & ANTI-ALLERGY AGENTS IN MEDICINAL CHEMISTRY, Bd. 9, Nr. 2, 1. Juni 2010 (2010-06-01), Seiten 142-149, XP009170173, BENTHAM SCIENCE PUBLISHERS LTD, BUSSUM, NL ISSN: 1871-5230
- Anonymous: "Eusolex T-PRO", Rona , XP002698586, Gefunden im Internet: URL:http://85.238.144.18/rona/EusolexTPRO_ brochure.pdf [gefunden am 2013-06-11]
- Anonymous: "SOLARINE III", Innovadex , März 2013 (2013-03), XP002698587, Gefunden im Internet: URL:http://www.innovadex.com/documents/117 3993.pdf?bs=1025&b=222594&st=20 [gefunden am 2013-06-11]

## Beschreibung

Die Erfindung betrifft ein Kosmetikum, das einen biologischen Naturstoffkomplex neben weiteren gut hautverträglichen Stoffen enthält.

Von der kosmetischen Industrie werden ständig neue Produkte auf den Markt gebracht, die in vielen Fällen eine Vielzahl synthetisch erzeugter Rohstoffe enthalten, die bei Anwendern mit empfindlicher Haut, manchmal schon bei den ersten Anwendungsversuchen, oft aber auch bei dauerhaftem Gebrauch zu Hautirritationen führen. Zwar sind in den letzten Jahren verstärkt auch natürliche Stoffe in Form von Pflanzenextrakten in Kosmetika angeboten und eingesetzt worden. Diese Produkte zeigen jedoch in den meisten Fällen nur eine ganz bestimmte Wirkungsrichtung, was nicht immer ausreichend ist.

So offenbart zum Beispiel die FR 2863163 allein eine Verbesserung von trockener Haut durch Einsatz eines Gemisches von Perlenextrakt und Extrakten von Baumfarn der Gattung Cyathea.

Aus BMC Complementary and Alternative Medicine 2010, 10:15 ist der Einsatz von Blechnum orientale als Antioxidant und antibakterielles Mittel bekannt, wobei die Extrakte mit Methanol gewonnen werden.
In einem Review fassen Philips et al in Anti-Inflamm & Anti- Allergy Agents in Med. Chemistry, 2010, 9, 142-149 Forschungsergebnisse zu Polypodium leucotomos gegen Hautalterung und -krebs zusammen.
Die FR 2696075A1 offenbart die Verwendung von Ecdysteroiden u.a. zur Verstärkung der Feuchtigkeitsbarriere der Haut, wobei die Methanol-Extrakte u.a. auch aus Polypodium vulgare gewonnen werden.
Die DE 10 2007 005 186A1 beschreibt ein Metlloxidgemisch aus TiO₂, MnO₂, Al₂O₃ als UV-Schutzmittel. In der DE 202006019183U1 wird ein Hautpflegeöl aus Sanddornfruchtfleisch-Öl, Vitamin E-acetat und einem weiteren Öl/Wachs offenbart. Die FR 2648347A1 betrifft ein Gemisch aus poly-ungesättigten Omega-3-Fettsäuren, Vitamin E und unverseifbaren pflanzlichen Fettstoffen, darunter auch unter mehreren anderen Vocanga.
Aus der FR 2768621 A1 ist die kosmetische Verwendung von verschiedenen Rosenblütenblätter-Extrakten bekannt

Der Erfindung liegt die Aufgabe zugrunde, Hautirritationen bei einem dauerhaft angewendeten kosmetischen Produkt weitgehend zu vermeiden und dabei eine deutlich verbesserte Hautfeuchtigkeit neben antiradikalischen, lichtschützenden und weiteren Wirksamkeiten zu erzielen.

Erfindungsgemäß bereitgestellt wird ein Kosmetikum mit biologischem Naturstoffkomplex, umfassend den Naturstoffkomplex, bestehend aus
- einem Extrakt von einem oder mehreren Farnen, ausgewählt unter Polypodium vulgare, Polypodium leucotomos, Dryopteris crassirhizoma, Matteuccia struthoptheris, Pteridium aquilinum, Blechnum orientale, Osmunda regalis und Polypodium aureum und Gemischen davon, wobei der Extrakt ein Extrakt der Gesamtpflanze mit mehrwertigen Alkoholen bei 15 - 25°C ist,
- einem teilchenförmigen anorganischen Oxidgemisch aus TiO₂, Al₂O₃ und MnO₂ und
- einem Gemisch aus Sanddorn-Fruchtfleischöl und Extrakten von Vocanga und Wildrosenfrüchten
und weiteren kosmetischen Hilfs- und Begleitstoffen.

Der Naturstoffkomplex zeigt im Zusammenwirken aller Bestandteile eine feuchthaltende Wirksamkeit, die über die Summe der Einzelwirkungen hinausgeht.

Besonders vorteilhaft für Anwender mit empfindlicher Haut ist es, dass bei Einschränkung auf nur wenige unbedenkliche kosmetische Hilfsstoffe neben dem biologischen Naturstoffkomplex eine oftmalige wiederholte Anwendung des erfindungsgemäßen Kosmetikums über längere Zeiträume ohne Hautirritationen möglich ist.

So konnten beispielsweise bei dreimaliger täglicher Anwendung des Kosmetikums als Serum, Creme oder Gel oder in Kombinationen davon über 2 bis 8 Wochen keine Irritationen bei empfindlicher Haut beobachtet werden. Dies ist besonders vorteilhaft, da trotz des Fehlens organischer Sonnenschutzfilter, die oft für hautirritierende Wirkungen bei empfindlicher Haut sorgen, über den biologischen Naturstoffkomplex eine durchaus beachtliche UV-Filterwirkung und Filterwirkung für den sichtbaren Lichtbereich entfaltet wird. Die unerwartet hohe Feuchtigkeits-Rückhaltewirkung sorgt zusätzlich für einen ausgeglichenen Wasserhaushalt der exponierten Hautoberflächen.

Erfindungsgemäß vorteilhaft ist es, wenn der Anteil der Farnextrakte in dem Naturstoffkomplex sowohl in liposomal verkapselter Form als auch direkt und unverkapselt vorliegt. Ein bevorzugtes Verhältnis von Farnen, die sowohl einzeln als auch im Gemisch enthalten sein können, in liposomal verkapselter zu unverkapselter Form, liegt im Bereich von 65 bis 98 : 35 bis 2, bezogen auf das Gewicht der Farnextrakte. Ein besonders bevorzugter Bereich des Verhältnisses liegt bei 85 bis 98 : 15 bis 2.

Besonders bevorzugt ist ein Bereich von 65 bis 95 Gew.-% verkapseltes Gemisch von drei Farnextrakten im Verhältnis zu 35 bis 5 Gew.-% eines einzelnen unverkapselten Farnextraktes. Dabei besteht das Gemisch vorteilhaft aus Polypodium vulgare, Polypodium leucotomos und Polypodium aureum, und der unverkapselte Farn ist Polypodium leucotomos.

Eine weitere besonders bevorzugte Ausführungsform besteht in einem Gemisch von in Liposomen verkapselten Extrakten von Polypodium vulgaris und Polypodium leucotomos sowie unverkapseltem Extrakt von Polypodium vulgare. Dabei liegt der Anteil des unverkapselten Extraktes vorteilhaft im Bereich von 2 bis 10 Gew-%, bezogen auf das Gesamtgewicht der Farnextrakte.

Eine Erhöhung des Anteils unverkapselter Farne über 35 in dem Verhältnis führt zu einer verschlechterten Anti-Irritationswirkung.

Auch die unverkapselten Farne können als Gemisch mehrerer Farnarten vorliegen. Bei Gemischen liegt das Verhältnis der Farnextrakte untereinander normalerweise bei 1 : 1 : 1 (Dreiergemisch) oder entsprechend 1 : 1 (Zweiergemisch).

Bevorzugte Farnarten sind Polypodium vulgare, Osmunda regalis, Polypodium aureum und Polypodium leucotomos und Gemische davon, besonders bevorzugt Polypodium vulgare und Polypodium leucotomos.

Die Farnextrakte werden aus der Gesamtpflanze gewonnen einschließlich des Rhizoms. Die Extraktion erfolgt mir mehrwertigen Alkoholen wie z. B. Propylenglycol oder Butylenglycol. Bevorzugt ist Propylenglycol. Die Extraktionstemperatur liegt im Bereich von 15 - 25 °C.

Der Anteil der Farnextrakte im Kosmetikum liegt im Bereich von 0,1 bis 8 Gew.-%, bezogen auf das Gesamtgewicht des Kosmetikums.

Als Material zur Bildung der Liposome sind beispielsweise Phospholipide geeignet. Als Phospholipide können z. B. eingesetzt werden Phosphatidylcholin, Phosphatidylethanolamin, Phosphatidylinositol, Phosphatidylserin, Phosphatidsäure und Lysolecithine sowie Gemische davon. Bekannte Produkte sind beispielsweise Phoslipon® oder NAT®.

Weitere Verkapslungsmaterialien können bestehen aus Kollagenmatrices, cyclischen Oligosacchariden, Cyclodextrinen wie alpha-, beta-, HP-beta-, partiell methyliertes beta- oder gamma-Cyclodextrin, Cellulose, Gelatine oder Wachsmatrices.

Für den Fall der Liposome so sind diese bekanntlich vollständig geschlossene Lipid-Bilayer-Membranen, die ein wässriges Volumen eingeschlossen enthalten. Liposome können unilamellare Vesikel sein (die eine Einzelmembran-Bilayer besitzen) oder multilamellare Vesikel (Onion-ähnliche Strukturen), gekennzeichnet durch Mehrfachmembran-Bilayer, von denen jede von der nächsten durch eine wässrige Schicht getrennt ist.

Liposome aus Phospholipiden sind das bevorzugte Verkapselungsmaterial.

Es ist weiterhin bevorzugt, dass das Gemisch aus Vocanga/Wildrosen-Extrakten und das Sanddorn-Fruchtfleischöl in Liposomen verkapselt sind.

Ein bevorzugtes zu verkapselndes Produkt auf dieser Basis ist Dicaprylyl Carbonate & Cocos Nucifera Oil & Hippophae Rhamnoides Fruit Oil & Rosa Canina fruit Extract & Voacanga Africana Seed Extract, wie z. B. Solarine III von Greentech, St. Beauzire, Frankreich.

Erfindungsgemäße Hilfs- und Begleitstoffe können solche sein, wie sie üblicherweise in kosmetischen Zusammensetzungen verwendet werden, wie z. B. Wasser, Konservierungsmittel, Farbstoffe, Farbpigmente, Duftstoffe, Verdikkungsmittel, ein- oder mehrwertige Alkohole, Ester, Elektrolyte, Gelbildner, polare und unpolare Öle, Polymere, Copolymere, Emulgatoren, Stabilisatoren.

Vorteilhaft für die vorliegende Erfindung sind solche Hilfsstoffe, die möglichst unbedenklich und irritationsfrei für empfindliche Haut sind. Dazu gehören Wasser, Gelbildner aus Naturstoffen wie Agar-Agar, Guar Gum, Carrageenan oder Xanthan Gum, weiterhin pflanzliche Öle, Shea Butter, Kakaobutter, Lanolin. Auch Ester oder Ether können eingesetzt werden, wie z. B. solche auf Milch- oder Zuckerbasis und z. B. solche auf Cetiol-Basis.

Weitere Zusatzstoffe können sein Hyaluronsäure oder Derivate davon, Glycerin, Lactose, Allantoin, weitere pflanzliche Wirkstoffe, Ubichinon, Biotin, Flavonoide und Isoflavonoide, β-Carotin, Vitamine und Vitaminderivate und andere. Bevorzugt sind Allantoin, Hyaluronsäure, Tocopherylacetat, -palmitat und -linoleat, Tocopherylacetat oder -succinat und Biotin.

Besonders bevorzugte Zusatzstoffe sind Vitamine wie Vitamin A und/oder Vitamin E sowie deren Derivate, Hyaluronsäure und weitere pflanzliche Wirkstoffe wie z. B. ein Passionsblumenextrakt der Blüten von Passiflora incarnata oder Acerola-Fruchtextrakt (Malphigia punicifolia). Die Pflanzenextrakte werden mit mehrwertigen Alkoholen wie z.B. Propylenglycol bei 15 - 18 °C gewonnen.

Der erfindungsgemäße Naturstoffkomplex ist kein Komplex im rein chemischen Sinne, sondern ein Gemisch verschiedener Substanzen, deren Zusammenwirken eine nicht vorhersehbare Wirkung zeigt. Der Komplex geht damit über die Summe der Einzelwirkungen der Bestandteile hinaus.

Die Erfindung betrifft auch die Verwendung eines Kosmetikums mit biologischem Naturstoffkomplex, umfassend den Naturstoffkomplex, bestehend aus
- einem Extrakt von einem oder mehreren Farnen, ausgewählt unter Polypodium vulgare, Polypodium leucotomos, Dryopteris crassirhizoma, Matteuccia struthoptheris, Pteridium aquilinum, Blechnum orientale, Osmunda regalis und Polypodium aureum
- einem teilchenförmigen anorganisches Oxidgemisch aus TiO₂, Al₂O₃ und MnO₂ und
- einem Gemisch aus Sanddorn-Fruchtfleischöl und Extrakten von Vocanga-Samen und Wildrosenfrüchten
   und weiteren kosmetischen Hilfs- und Begleitstoffen
zur Erhöhung des Feuchtigkeitsgehaltes der Haut.

Besonders bevorzugt sind Gemische von Polypodium vulgare und Polypodium leucotomos in Liposomen und zusätzlich ein Anteil von Polypodium vulgare unverkapselt, wobei der unverkapselte Anteil im Bereich von 5-35 Gew-% beträgt, bezogen auf das Gesamtgemisch der Farnextrakte.

Ein weiterer Gegenstand der Erfindung ist die Verwendung des o. g. Kosmetikums mit dem biologischen Naturstoffkomplex zur Vermeidung von Hautirritationen bei wiederholten (zwei bis vier) täglichen Anwendungen in Zeiträumen von 2 bis 8 Wochen.

Die Erfindung soll nachstehend durch Beispiele näher erläutert werden. Alle Angaben erfolgen in Gewichtsprozent, sofern nichts anderes angegeben ist.

### Beispiel 1 Gelcreme (O/W Emulsion)

| **Phase 1** | |
|---|---|
| Wasser | q.s. ad 100 |
| Carbopol 940 (Gel) | 0,6 |
| Hyaluronsäure | 0,03 |
| Glycerin | 2,0 |
| Propylenglycol | 2,5 |
| | |

| **Phase 2** | |
|---|---|
| Cetearyl Isononanoate (als Cetiol® SN) | 0,5 |
| Konservierungsmittel | 1,0 |
| Vitamin E | 0,5 |
| Kakaobutter | 0,8 |
| Vitamin A | 0,02 |
| Titanium Dioxide, Alumina, Manganese Dioxide (als Eusolex® T-PRO) | 0,03 |

| **Phase 3** | |
|---|---|
| Triethanolamine | 0,8 |
| Dicaprylyl Carbonate & Cocos Nucifera Oil & Hippophae Rhamonoides Fruit Oil & Rosa Canina Fruit Extract & Vocanga Africana Seed Extract (als Solarine III) | 0,5 |
| Farnkomplex aus Polypodium vulgare und Polypodium leucotomos (verkapselt) | 1,5 |
| Acerola Fruchtextrakt (als Aceromine®*) | 0,6 |

| | |
|---|---|
| *(Water& Malpighia Punicifolia Fruit Extract& Alcohol Denat& Glycerin& Lecithin& Guar& Hydroxypropyltrimonium Chloride&Phenoxyethanol& Tocopheryl Acetate& Benzophenone-4) | |

| | |
|---|---|
| Parfüm | 0,3 |
| Cetiol® Soft | 0,5 |
| Pearl Extract (als Perlecine® (Greentech SA)) | 0,5 |
| Polypodium vulgare Extrakt (unverkapselt) | 0,1 |
| Titanium Dioxide& Alumina& Manganese Dioxide (als Eusolex® T-PRO) | 0,05 |

Die Phase 1 wird auf 65 °C erhitzt und dabei gerührt. Separat wird die Phase 2 auf 65 °C erhitzt, dabei ebenfalls gut gerührt. Die Phase 1 wird unter Rühren zur Phase 2 hinzugeben. Danach lässt man auf 35 - 40 °C abkühlen.

Die Bestandteile der Phase 3 werden dem Gemisch unter Rühren bis zum Erreichen einer homogenen Gelcreme zugesetzt.

### Beispiel 2 Reichhaltige Creme (W/O Emulsion)

| **Phase 1** | |
|---|---|
| Wasser | q.s. ad 100 |
| NaCl | 0,9 |
| Glycerin | 4,0 |
| | |

| **Phase 2** | |
|---|---|
| naturbasierter Gelbildner (als Akil En90, A. Muller) | 0,6 - 1,0 |
| W/O Emulgator (als Pioneer® 6130) | 1,8 |
| Hydrogenated Castor Oil (als Cutina® HR) | 1,0 |
| Ethylhexyl Stearate (als Cetiol® 686) | 8,0 |
| Vitamin E | 0,3 |
| Isohexadecane (als Aramol® HD) | 9,5 |
| Phenoxyethanol & Ethylhexylglycerin (als Euxyl® 9010) | 1,0 |
| Vitamin A | 0,02 |
| Titanium Dioxide / Alumina / Manganese Dioxide (als Eusolex® T-PO) | 0,2 |
| | |

| **Phase 3** | |
|---|---|
| Acerola Fruchtextrakt (als Aceromine®) | 0,5 |
| Farnkomplex von Beispiel 1 (verkapselt) | 1,0 |
| Parfüm | 0,5 |
| Solarine III | 0,2 |
| Pearl Extract (als Perlecine®, Greentech FR) | 0,5 |
| Hyaluronic Acid & Jojoba Oil (als Gelnyperm) | 2,0 |
| Polypodium vulgare Extrakt (unverkapselt) | 0,03 |

Die Phase 1 wird unter Rühren bei 500 - 800 U/min und 75 °C hergestellt. Die Phase 2 wird ebenfalls unter Rühren bei 75 °C - 85 °C und ca. 500 - 800 U/min hergestellt, danach wird für 10 Minuten bei ca. 3000 U/min homogenisiert. Phase 2 wird zu Phase 1 gegeben und homogenisiert. Nach Abkühlen auf 40 °C werden nacheinander die Bestandteile der Phase 3 hinzugegeben und homogenisiert. Dabei wird die Temperatur von ca. 30 °C erreicht.

Bei sofortiger Verteilung auf der Haut erzielt die reichhaltige Creme einen nachhaltigen Film.

### Beispiel 3 Augencreme O/W Emulsion

| **Phase 1** | |
|---|---|
| Wasser | q.s. ad 100 |
| Propylenglycol | 3,0 |
| Glycerin | 1,5 |
| Acrylates / C10-30 Alkyl Acrylate Crosspolymer (als Pemulen TR 1) | 0,2 |
| | |

| **Phase 2** | |
|---|---|
| Glyceryl Stearate & Ceteareth-12 & Cetearyl Alcohol & Cetyl Palmitate (als Emulgade® SE) | 6,0 |
| Cetearyl Alcohol (als Lanette® O) | 1,5 |
| Makademia-Nußöl | 1,0 |
| Jojobaöl | 2,0 |
| Decyl Oleate (als Cetiol® V) | 3,0 |
| Ethylhexyl Stearate (als Cetiol® 868) | 1,0 |
| Vitamin E | 0,5 |
| Vitamin A | 0,02 |
| | |

| **Phase 3** | |
|---|---|
| Phenoxyethanol & Ethylhexylglycerin (als Euxyl® PE 9010) | 1,0 |
| Hyaluronsäure | 0,01 |
| | |

| **Phase 4** | |
|---|---|
| Triethanolamine | 0,25 |
| | |

| **Phase 5** | |
|---|---|
| Cyclomethicone or Cyclopenta siloxane (als Mirasil® CM 5) | 2,0 |
| Podia Extract | 0,5 |
| Acerola Fruchtextrakt (als Aceromine®) | 0,5 |
| Farnkomplex gemäß Beispiel 1 (in Liposomen) | 1,0 |
| Ethanol | 2,0 |
| Perlextrakt (Mother of Pearl Extract) (als Perlecine®, Greentech FR) | 0,5 |
| Kamille | 1,0 |
| Eusolex® T-PRO | 0,05 |
| Solarine III | 0,1 |
| Polypodium leucotomos Extrakt (unverkapselt) | 0,1 |

Die Phase 1 wird unter Rühren bei 65 °C hergestellt. Phase 2 wird ebenfalls bei 65 °C unter Rühren und bei anschließendem Homogenisieren hergestellt. Danach wird Phase 1 der Phase 2 zugesetzt und das Gemisch bei fortlaufendem Rühren auf ca. 40 °C abgekühlt. Dem Gemisch wird die Phase 3 zugesetzt, danach die Phase 4, und es wird ca. 10 Minuten homogenisiert. Bei einer Temperatur von ca. 35 °C werden die Bestandteile der Phase 5 nacheinander zugegeben. Im Anschluss daran wird 10 Minuten homogenisiert.

### Beispiel 4 Vergleichsversuche

### 4.1 Feuchtigkeit

In Bezug auf die Anreicherung der Haut mit Feuchtigkeit wurden Feuchtigkeitsmessungen mit einem Corneometer CM 825 (Conrage & Khazaha, Köln, Deutschland) durchgeführt. Die Messungen erfolgten bei 55 % rel. Luftreuchte (20 °C). Bei 16 Probanden mit empfindlicher Mischhaut wurden vier unterschiedliche Gelcremes aufgetragen.

Die Basisgelcremes bestanden aus

| | |
|---|---|
| 0,6 % | Akil En90 (naturbasierter Gelbildner) |
| 0,03 % | Hyaluronsäure |
| 2,0 % | Glycerin |
| 2,5 % | Propylenglycol |
| 0,5 % | Cetostearyl Isononanoate |
| 1,0 % | Konservierungsmittel |
| 0,5 % | Tocopherylacetat |
| 0,8 % | Kakaobutter |
| 0,02 % | Retinylacetat |
| 0,8 % | Triethanolamin |
| 2,0 % | Cetiol® Soft |
| q.s. ad 100% | Wasser |

Zu den Basiscremes wurden hinzugesetzt unter entsprechender Verringerung des Anteiles an Wasser:

| | | |
|---|---|---|
| Creme 4A: | 1,0 % Farngemisch, bestehend aus Polypodium vulgare, Polypodium leucotomos und Polypodium aureus verkapselt in Liposomen und 0,1 % Polypodium vulgare Extrakt (unverkapselt). | |
| Creme 4B: | 0,08 % TiO₂, MnO₂ Al₂O₃ (Eusolex® T-PRO) | |
| Creme 4C: | 0,5 % Solarine III | (Dicaprylyl Carbonate & Cocos Nucifera Oil & Hippophae Rhamonoides Fruit Oil & Rosa Canina Fruit Extract & Vocanga Africana Seed Extract) |
| Creme 4D: | 1,0 % Farngemisch wie Creme 4A und 0,1 % Polypodium vulgare (unverkapselt) | |
| | 0,08 % Eusolex® T-PRO | |
| | 0,5 % Solarine III | |

Die gemittelten Ergebnisse sind als prozentuale Feuchtigkeitsverbesserung in der folgenden Tabelle enthalten.

| Messzeit nach Stunden | Creme 4A | Creme 4B | Creme 4C | Creme 4D (Erfindung) |
|---|---|---|---|---|
| 0,5 | 31 | 8 | 22 | 62 |
| 3 | 28 | 6 | 11 | 53 |
| 9 | 16 | 3 | 6 | 29 |
| 24 | 8 | - | 2 | 18 |

Die erfindungsgemäße Gelcreme 4D zeigt nach allen Messzeiten eine deutlich höhere Feuchthaltewirkung als die Gesamtheit der Einzelergebnisse.

### 4.2 Irritation

Die Gelcreme 4D vom Beispiel 4.1 wurde gegen eine UV-Creme vom Markt mit einem SPF 15 bei 12 Probanden mit empfindlicher Heut getestet.

Beide Cremes wurden dreimal täglich auf jeweils einen Arm aufgetragen mit einer Menge von etwa 2,5 mg/cm². Nach 5, 10 und 14 Tagen wurde jeweils eine Stunde nach dem Auftragen an einem Tag visuell von einem Panel von 3 Testleitern die Hautrötung beurteilt.

Bei ca. 40 % der Probanden war nach 10 Tagen eine leichte Hautrötung auf Stellen mit der Creme vom Markt erkennbar. Nach 14 Tagen wurde eine mittlere Hautrötung bei diesen Probanden festgestellt.

Für die Creme 4D der Erfindung war keinerlei Veränderung in Bezug auf Hautrötung zu erkennen.

## Patentansprüche

1. Kosmetikum mit biologischem Naturstoffkomplex, umfassend den Naturstoffkomplex, bestehend aus
- einem Extrakt von einem oder mehreren Farnen, ausgewählt unter Polypodium vulgare, Polypodium leucotomos, Dryopteris crassirhizoma, Matteuccia struthoptheris, Pteridium aquilinum, Blechnum orientale, Osmunda regalis und Polypodium aureum, wobei der Extrakt ein Extrakt der Gesamtpflanze mit mehrwertigen Alkoholen bei 15 - 25°C ist,
- einem teilchenförmigen anorganisches Oxidgemisch aus TiO₂, Al₂O₃ und MnO₂ und
- einem Gemisch aus Sanddorn-Fruchtfleischöl und Extrakten von Vocanga-Samen und Wildrosenfrüchten
und weiteren kosmetischen Hilfs- und Begleitstoffen.

2. Kosmetikum nach Anspruch 1, **dadurch gekennzeichnet, dass** die Farne ausgewählt sind unter Polypodium vulgare, Osmunda regalis, Polypodium aureum und Polypodium leucotomos und Gemischen davon, insbesondere unter Polypodium vulgare, Polypodium leucotomos und Gemischen davon.

3. Kosmetikum nach Anspruch 1 oder 2, **dadurch gekennzeichnet, dass** die Farne in verkapselter Form in Liposomen und in unverkapselter Form nebeneinander in dem Komplex vorliegen.

4. Kosmetikum nach Anspruch 3, **dadurch gekennzeichnet, dass** das Verhältnis von Farnen in verkapselter Form zu unverkapselten Farnen im Bereich von 70 - 98 : 30 - 2 beträgt, bezogen auf das Gewicht.

5. Kosmetikum nach einem der Ansprüche 1 bis 4, **dadurch gekennzeichnet, dass** in dem teilchenförmigen anorganischen Material das TiO₂ mit MnO₂ beschichtet ist.

6. Kosmetikum nach einem der Ansprüche 1 - 5, **dadurch gekennzeichnet, dass** das Gemisch aus Extrakten und Sanddorn-Fruchtfleischöl in Liposomen verkapselt ist.

7. Kosmetikum nach einem der Ansprüche 1 - 6, **dadurch gekennzeichnet, dass** der Anteil der Farne im Bereich von 0,1 - 8 Gew.-% liegt, bezogen auf das Gesamtgewicht des Kosmetikums.

8. Kosmetikum nach einem der Ansprüche 1 - 7, **dadurch gekennzeichnet, dass** der Anteil des Oxidgemisches im Bereich von 0,1 - 0,9 Gew.-% liegt, bezogen auf das Gesamtgewicht des Kosmetikums.

9. Kosmetikum nach einem der Ansprüche 1 - 8, **dadurch gekennzeichnet, dass** der Anteil des Extraktgemisches zusammen mit dem Öl im Bereich von 0,2 bis 5 Gew.-% liegt, bezogen auf das Gesamtgewicht des Kosmetikums.

10. Kosmetikum nach einem der Ansprüche 1 - 9, **dadurch gekennzeichnet, dass** es weiterhin einen oder mehrere Pflanzenextrakte enthält, ausgewählt unter Blütenextrakten von Passiflora incarnata, Fruchtextrakten von Acerola (Malphigia punicifolia) und Gemischen davon.

11. Nicht-therapeutische Verwendung eines Kosmetikums nach Anspruch 1 zur Erhöhung des Feuchtigkeitsgehaltes der Haut.

## Claims

1. A cosmetic product with biological natural material complex comprising the natural material complex, consisting of
- an extract from one or more ferns selected from Polypodium vulgare, Polypodium leucotomos, Dryopteris crassirhizoma, Matteuccia struthoptheris, Pteridium aquilinum, Blechnum orientale, Osmunda regalis and Polypodium aureum, wherein the extract is an extract of the entire plant with polyhydric alcohols at 15 - 25°C,
- a particulate inorganic oxide mixture of titanium dioxide, aluminum oxide and manganese dioxide, and
- a mixture of sea buckthorn fruit oil and extracts of Vocanga seeds and wild rose petals;
and other cosmetic auxiliary and ancillary materials.

2. The cosmetic product according to Claim 1, **characterized in that** the ferns are selected from Polypodium vulgare, Osmunda regalis, Polypodium aureum and Polypodium leucotomos and mixtures thereof, in particular from Polypodium vulgare, Polypodium leucotomos and mixtures thereof.

3. The cosmetic product according to Claim 1 or 2, **characterized in that** the ferns exist in an encapsulated form in liposomes and in a non-encapsulated form next to one another in the complex.

4. The cosmetic product according to Claim 3, **characterized in that** the ratio of ferns in an encapsulated form to non-encapsulated ferns is in the range of 70 - 98:30 - 2, based on the weight.

5. The cosmetic product according to any one of Claims 1 to 4, **characterized in that** in the particulate inorganic material the TiO₂ is coated with MnO₂.

6. The cosmetic product according to any one of Claims 1 - 5, **characterized in that** the mixture of extracts and sea buckthorn fruit oil is encapsulated in liposomes.

7. The cosmetic product according to any one of Claims 1 - 6, **characterized in that** the proportion of the ferns is in the range of 0.1 to 8% by wt., based on the total weight of the cosmetic product.

8. The cosmetic product according to any one of Claims 1 - 7, **characterized in that** the proportion of the oxide mixture is in the range of 0.1 to 0.9% by wt., based on the total weight of the cosmetic product.

9. The cosmetic product according to any one of Claims 1 - 8, **characterized in that** the proportion of the extract mixture together with the oil is in the range of 0.2 to 5% by wt., based on the total weight of the cosmetic product.

10. The cosmetic product according to any one of Claims 1 - 9, **characterized in that** it further contains one or more plant extracts selected from flower extracts of Passiflora incarnata, fruit extracts of Acerola (Malphigia punicifolia) and mixtures thereof.

11. Non-therapeutic use of a cosmetic product according to Claim 1 for increasing the moisture content of the skin.

## Revendications

1. Cosmétique avec complexe biologique en matière naturelle, comprenant le complexe en matière naturelle, composé de
- un extrait d'une ou de plusieurs fougères, sélectionnées parmi Polypodium vulgare, Polypodium leucotomos, Dryopteris crassirhizoma, Matteuccia struthoptheris, Pteridium aquilinum, Blechnum orientale, Osmunda regalis et Polypodium aureum, l'extrait étant un extrait de la plante complète avec des polyalcools à 15 - 25°C,
- un mélange d'oxydes inorganique particulaire en TiO₂, Al₂O₃ et MnO₂ et
- un mélange d'huile de pulpe de baies d'argousier et d'extraits de semences de vocanga et de fruits de rose musquée
et d'autres substances auxiliaires et annexes cosmétiques.

2. Cosmétique selon la revendication 1, **caractérisé en ce que** les fougères sont sélectionnées parmi Polypodium vulgare, Osmunda regalis, Polypodium aureum et Polypodium leucotomos et leurs mélanges, en particulier parmi Polypodium vulgare, Polypodium leucotomos et leurs mélanges.

3. Cosmétique selon la revendication 1 ou 2, **caractérisé en ce que** les fougères se présentent de façon juxtaposée dans le complexe sous forme encapsulée dans des liposomes et sous forme non encapsulée.

4. Cosmétique selon la revendication 3, **caractérisé en ce que** le rapport des fougères sous forme encapsulée aux fougères non encapsulées est compris dans la plage de 70 - 98 : 30 - 2, rapporté au poids.

5. Cosmétique selon une des revendications 1 à 4, **caractérisé en ce que**, dans le matériau inorganique particulaire, le TiO₂ est revêtu de MnO₂.

6. Cosmétique selon une des revendications 1 à 5, **caractérisé en ce que** le mélange d'extraits et d'huile de pulpe de baies d'argousier est encapsulé dans des liposomes.

7. Cosmétique selon une des revendications 1 à 6, **caractérisé en ce que** la proportion des fougères se situe dans la plage de 0,1 - 8 %-poids, rapportée au poids total du cosmétique.

8. Cosmétique selon une des revendications 1 à 7, **caractérisé en ce que** la proportion du mélange d'oxydes se situe dans la plage de 0,1 - 0,9 %-poids, rapportée au poids total du cosmétique.

9. Cosmétique selon une des revendications 1 à 8, **caractérisé en ce que** la proportion du mélange d'extraits avec l'huile se situe dans la plage de 0,2 - 5 %-poids, rapportée au poids total du cosmétique.

10. Cosmétique selon une des revendications 1 à 9, **caractérisé en ce qu'**il contient également un ou plusieurs extraits de plantes, sélectionné(s) parmi des extraits de fleurs de Passiflora incarnata, des extraits de fruits d'acérola (Malphigia punicifolia) et leurs mélanges.

11. Utilisation non thérapeutique d'un cosmétique selon la revendication 1 pour augmenter la teneur en humidité de la peau.
